# EUROPEAN PATENT APPLICATION

(11) **EP 4 737 006 A1**
(43) Date of publication of application: **06.05.2026**
(21) Application number: 25207689.8
(22) Date of filing: 09.10.2025
(51) Int. Cl.: B01L 3/00

(54) **ASSAY DEVICE AND TARGET MOLECULE TESTING METHOD**

(30) Priority: 30.10.2024 JP 2024191069
(71) Applicant: DENSO CORPORATION, Kariya-city, Aichi-pref., 448-8661 (JP)
(72) Inventor: NAKAMURA, Kazuya, Kariya-city, Aichi-pref., 448-8661 (JP); NAKAGAWA, Kazuhisa, Kariya-city, Aichi-pref., 448-8661 (JP); KADOI, Masaru, Kariya-city, Aichi-pref., 448-8661 (JP)
(74) Representative: Winter, Brandl - Partnerschaft mbB

(57) **Abstract**

An assay device configured to detect a target molecule includes: a pretreatment unit (100) having a circulation channel (105) which is a tubular passage through which a liquid circulates; and a bubble remover (112, 112a, 112b, 112c, 112d, 112e, 112f) configured to remove or capture bubbles mixed in the liquid circulating in the circulation channel by utilizing a difference in properties between liquid and gas. The target molecule is bound with a detection material for detecting the target molecule, and a cleaning is performed with a washing solution to separate unreacted substance that is not bound, as a pretreatment for detecting the target molecule.

## Description

The present disclosure relates to an assay device and a target molecule testing method.

A test called POCT (Point of Care Testing) is known, which shortens the time from when a sample is collected to when test results are obtained. Many of the assay devices used in POCT are based on the principles of immunoassay methods. Some immunoassay methods require a binding free (BF) separation, which is the separation of unreacted substances.

JP 2013-536952 A discloses an assay cartridge in which binding of an analyte to a binder and subsequent washing are performed within a channel. In the technique of JP 2013-536952 A, a liquid sample containing an analyte is introduced into a cartridge through an inlet. In the technique of JP 2013-536952 A, the channel is a sealed system.

In the technique disclosed in JP 2013-536952 A, air bubbles may be mixed into the sealed channel when the liquid sample is introduced. The details will be described as follows. If the analyte is trapped in the air bubbles, the air bubbles will inhibit the binding of the analyte and subsequent washing, reducing the accuracy of detection of the analyte. Furthermore, the technique disclosed in JP 2013-536952 A does not take into consideration stirring of the fluid within the channel. This can result in insufficient binding of the analyte within the channel.

One objective of this disclosure is to provide an assay device and a target molecule testing method to make it easier for a target molecule to bind within a channel, when the target molecule is bound to a binding target and washed within the channel, and to reduce the inhibition of binding and washing of target molecule due to the inclusion of bubbles in the channel.

The above objective is achieved by the combination of features described in the independent claims, and the dependent claims define further advantageous specific examples of the disclosure. The reference signs in parentheses in the claims indicate a correspondence relationship with specific means described in the embodiments described later as an aspect, and do not limit the technical scope of the present disclosure.

In order to achieve the above-mentioned objective, an assay device of the present disclosure is used for detecting a target molecule, and includes a pretreatment unit in which the target molecule is bound to a detection material for detecting the target molecule, as a pretreatment for detecting the target molecule, and washing is performed using a washing solution to separate unreacted substance that has not been bound. The binding and washing are performed in the pretreatment unit. The pretreatment unit includes: a circulation channel which is a tubular passage for circulating a liquid used for the binding and the washing; and a bubble remover to remove or capture air bubbles from the liquid in the circulation channel by utilizing a difference in properties between liquid and gas.

In order to achieve the above-mentioned objective, a target molecule testing method of the present disclosure is used for detecting a target molecule, and includes: a pretreatment process for binding the target molecule to a detection material for detecting the target molecule as a pretreatment for detecting the target molecule; and a washing process for separating unreacted substance not bound in the binding with a washing solution. The pretreatment includes: a circulation process for circulating a liquid in a circulation channel which is a tubular passage for circulating the liquid used for the binding and washing, where the binding and washing are performed; and a removal process for removing or capturing bubbles from the liquid in the circulation channel by a bubble remover to remove or capture bubbles by utilizing a difference in properties between liquid and gas.

Accordingly, the binding with the detection material and the washing of unreacted substance, as pretreatment for detecting the target molecule, are performed in the circulation channel. Since the liquid is circulated in the circulation channel, the target molecule and the detection material in the circulation channel are more likely to be agitated by this circulation. This increases the chance of the target molecule binding to the detection material, making it possible to increase the rate at which the target molecule is bound to the detection material. Since the circulation channel is tubular, there is a possibility that bubbles mixed in the liquid in the circulation channel may circulate within the circulation channel together with the target molecule and the detection material. In contrast, according to the above configuration, the bubbles are removed from the liquid or captured by the bubble remover. By utilizing the difference in properties between liquid and gas, it is possible to separate the liquid from the bubbles. Thus, the bubble remover makes it possible to remove or capture bubbles from the liquid. Therefore, the bubble remover reduces the continuous contact of bubbles with the target molecule and the detection material, which is less likely to inhibit the binding and the washing. As a result, even when binding and washing of a target molecule and a binding target are performed within a channel, it is possible to facilitate binding of the target molecule within the channel while reducing the hindrance of binding and washing of the target molecule due to bubbles entering the channel.
FIG. 1 is a diagram showing a schematic configuration of a testing system.
FIG. 2 is a diagram showing a schematic configuration of a cartridge.
FIG. 3 is a schematic view showing a first example of a trap mechanism.
FIG. 4 is a schematic view showing a second example of a trap mechanism.
FIG. 5 is a schematic view showing a third example of a trap mechanism.
FIG. 6 is a schematic view showing a fourth example of a trap mechanism.
FIG. 7 is a schematic view showing a fifth example of a trap mechanism.
FIG. 8 is a schematic view showing a sixth example of a trap mechanism.
FIG. 9 is a schematic view showing a sensor area according to a first embodiment.
FIG. 10 is a schematic view for explaining a function of a diaphragm.
FIG. 11 is a flowchart showing a detection-related process in the cartridge.
FIG. 12 is a graph showing a change in voltage over time, measured by a sensor chip.
FIG. 13 is a schematic view showing a sensor area according to a second embodiment.

Several embodiments for disclosure will be described with reference to the drawings. For convenience of explanation, portions having the same functions as those illustrated in the drawings used in the description among embodiments are assigned the same reference symbol, and descriptions of the same portions may be omitted. The descriptions of other embodiments may be referred to with respect to these portions given the same reference signs.

### (First Embodiment)

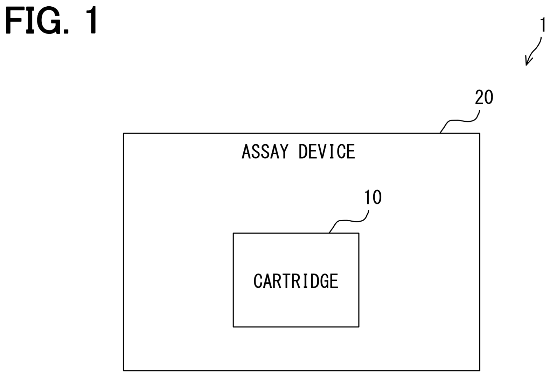

Hereinafter, a first embodiment of the present disclosure will be described with reference to the drawings. A testing system 1 shown in FIG. 1 is used to detect a target molecule as an analyte. The testing may simply involve detecting the presence of a target molecule, and may even involve quantifying the amount of the target molecule.

In the present disclosure, a target molecule to be detected refers to a molecule to be detected in a sample. A sample refers to any solution, substance, mixture, etc. that contains plural molecules and may include at least one target molecule. The target molecule may be of various types. Examples of target molecules include viruses, cells, antigens, proteins, sugar chains, small molecules, bacteria, antibodies, lipids, peptides, and nucleic acids. The target molecule may be a pathogen itself, such as a virus or bacterium, or may be a part of the pathogen, such as a protein or nucleic acid.

In the testing system 1 of the present disclosure, a complex is formed by binding a target molecule to a solid phase and a labeling substance, and the target molecule is tested based on a detection signal generated by the labeling substance. The solid phase is a material that facilitates the separation of target molecule from non-target substance. The solid phase may also be referred to as an immobilizing member. The solid phase may be any solid phase that can be circulated together with the liquid within the circulation channel 105 and that can be retained in a specific region within the circulation channel 105 by utilizing the properties of the solid phase. As the solid phase, it is preferable to use magnetic beads. The magnetic beads can be held in a specific region within the circulation channel 105 by magnetic force. In the following, the explanation will be continued by taking as an example a case where the magnetic beads are used as the solid phase. A binding element (hereinafter, referred to as a first binding element) for capturing a target molecule is immobilized on the magnetic beads that serve as the solid phase. This immobilization may be achieved by covalent bonding between the amino groups of the target molecule and the carboxyl groups of the magnetic beads.

The first binding element has reaction specificity for the target molecule. The first binding element may be an antibody or an aptamer. The antibody referred to here also includes antibody fragments and modified antibodies that have substantially the same reactivity as the antibody. The aptamer may be a nucleic acid aptamer or a peptide aptamer. The nucleic acid aptamer may be a DNA aptamer or an RNA aptamer. The nucleic acid aptamer may include an artificial nucleic acid. An aptamer is a nucleic acid molecule or peptide that specifically binds to a particular molecule. In this embodiment, the explanation will be continued taking as an example a case where an antibody is used as the first binding element. In the following, the complex of the solid phase and the first binding element is an antibody-attached magnetic bead.

The labeling substance facilitates detection of a target molecule. The labeling substance generates a detection signal, thereby facilitating detection of the target molecule bound by the binding element to which the labeling substance 50 is bound (hereinafter, referred to as the second binding element). The detection signal may be any signal that can be detected by a sensor. The detection signal can also be referred to as a signal. The detection signal may be ions, electric signals, heat, aggregation, fluorescence, dyes, and the like. The labeling substance may generate a detection signal by itself, or may be an enzyme, DNAzyme, or RNAzyme that functions as a catalyst to generate a detection signal. In this embodiment, the explanation will be continued by taking as an example a case where the labeling substance is an enzyme. In this embodiment, alkaline phosphatase (ALP) is used as the labeling substance. ALP catalyzes the chemical reaction of substrate and generates hydrogen ions as a detection signal. As a substrate, p-nitrophenyl phosphate may be used. When p-nitrophenyl phosphate and water are subjected to a hydrolysis reaction using ALP as a catalyst, p-nitrophenol, phosphate ions, and hydrogen ions are generated.

The second binding element is bound to the labeling substance that generates a detection signal. The second binding element has reaction specificity for the target molecule. The second binding element may be bound to the labeling substance by, for example, a covalent bond. The second binding element may be an antibody or an aptamer, as described for the first binding element. In this embodiment, an aptamer is used as the second binding element. In the following, the complex of the labeling substance and the second binding element will be described as an aptamer-enzyme fusion. That is, the target molecule forms a complex with the antibody-attached magnetic beads and the aptamer-enzyme fusion. The antibody-attached magnetic beads and the aptamer-enzyme fusion represent a detection material.

As shown in FIG. 1, the testing system 1 includes a cartridge 10 and an assay device 20. The cartridge 10 is a disposable component used in POCT to perform a particular test. The configuration of the cartridge 10 will be described later. The cartridge 10 is detachably attached to the assay device 20. The assay device 20 performs diagnosis and the like based on information obtained from the cartridge 10. The assay device 20 has a computer including, for example, a processor, a volatile memory, a non-volatile memory, an I/O, and a bus connecting these. The assay device 20 executes the above-mentioned diagnostic and other processes using this computer. The assay device 20 may include a user interface such as an operation input unit and a presentation device. The assay device 20 may include the cartridge 10.

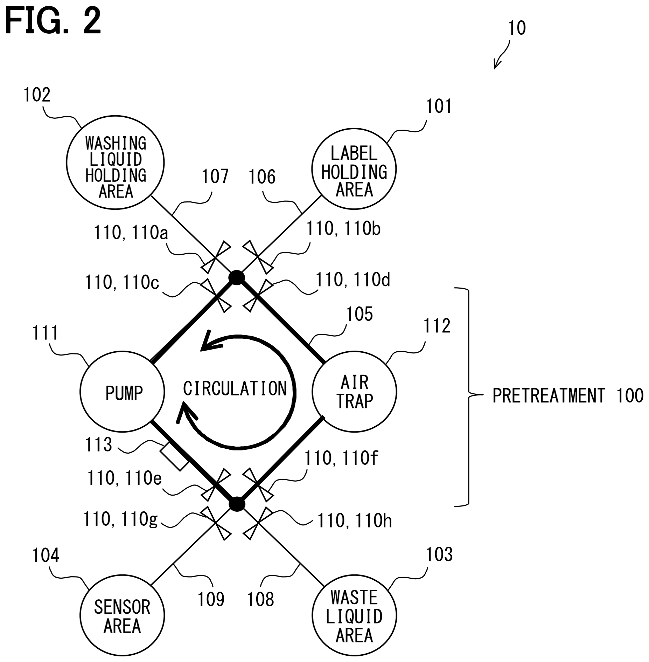

Next, the schematic configuration of the cartridge 10 will be described with reference to FIG. 2. As shown in FIG. 2, the cartridge 10 includes a label holding area 101, a washing liquid holding area 102, a waste liquid area 103, a sensor area 104, a circulation channel 105, a label introduction channel 106, a washing liquid introduction channel 107, a waste liquid channel 108, a sensor channel 109, a switching valve 110, a pump 111, an air trap 112, and a magnetic unit 113. The cartridge 10 may correspond to an assay device. The assay device 20 including the cartridge 10 may correspond to an assay device.

The label holding area 101 holds the aptamer-enzyme fusion, which is a complex of a labeling substance and a second binding element. The label holding area 101 holds a specified amount of buffer containing the aptamer-enzyme fusion. The specified amount is set as a predetermined value within the capacity of the circulation channel 105. The label holding area 101 may include, for example, a resin container having a depression for storing liquid.

The washing liquid holding area 102 holds the washing liquid. The washing liquid is a solution for washing away unreacted substances resulting from the binding of the target molecule, the antibody-attached magnetic beads and the aptamer-enzyme fusion. The washing liquid may be a washing buffer. The washing liquid holding area 102 may include, for example, a resin container having a depression for storing liquid. The amount of washing liquid held in the washing liquid holding area 102 may be set to allow multiple cleanings with the specified amount of washing liquid.

The waste liquid area 103 stores the waste liquid. The waste liquid includes liquid other than the complex of the target molecule and the antibody-attached magnetic beads (hereinafter, referred to as the target-beads complex) after the target molecule and the antibody-attached magnetic beads are bound to each other. The waste liquid includes liquid other than the complex of the target-beads complex and the aptamer-enzyme fusion (hereinafter referred to as the sandwich complex) after the target-beads complex and the aptamer-enzyme fusion are bound to each other. The waste liquid may be the washing liquid after washing. The waste liquid area 103 may include, for example, a resin container having a depression for storing liquid.

The sensor area 104 performs sensing based on a substance produced by the reaction between the aptamer-enzyme fusion of the sandwich complex and the reaction liquid. In this embodiment, the reaction liquid contains a substrate such as p-nitrophenyl phosphate. The sensor area 104 corresponds to a sensor. The sensor area 104 will be described in detail later.

The circulation channel 105 is a site where the binding and the washing are performed. In the circulation channel 105, the target molecule is bound to the antibody-attached magnetic beads and the aptamer-enzyme fusion, and the unbound and unreacted substances are washed away. The circulation channel 105 is a tubular passage to circulate the liquid for the bonding and washing. The circulation channel 105 may be made of, for example, a tubular resin. In this embodiment, a silicon tube is used as the circulation channel 105.

The label introduction channel 106 introduces a buffer containing the aptamer-enzyme fusion from the label holding area 101 to the circulation channel 105. The label introduction channel 106 may be made of, for example, a tubular resin. In this embodiment, a silicon tube is used as the label introduction channel 106.

The washing liquid introduction channel 107 is a flow path for introducing the washing liquid from the washing liquid holding area 102 to the circulation channel 105. The washing liquid introduction channel 107 may be made of, for example, a tubular resin. In this embodiment, a silicon tube is used as the washing liquid introduction channel 107.

The waste liquid channel 108 is a flow path for discharging the waste liquid from the circulation channel 105 to the waste liquid area 103. The waste liquid channel 108 may be made of, for example, a tubular resin. In this embodiment, a silicon tube is used as the waste liquid channel 108.

The sensor channel 109 is a flow path for the liquid between the sensor area 104 and the circulation channel 105. In this embodiment, a reaction liquid flows between the sensor area 104 and the circulation channel 105 through the sensor channel 109. The sensor channel 109 may be made of, for example, a tubular resin. In this embodiment, a silicon tube is used as the sensor channel 109.

The switching valve 110 is used to open and close the channel. For example, the switching valve 110 may compress and release a silicon tube serving as a channel, thereby opening and closing the channel. The switching valve 110 may be driven under the control of, for example, a microcomputer, an IC, or the like. The switching valve 110 may be controlled by the assay device 20 via a communication path that is electrically connected when the cartridge 10 is attached to the assay device 20. The switching valve 110 includes switching valves 110a to 110h.

The switching valve 110a is provided in the washing liquid introduction channel 107. As shown in FIG. 2, the switching valve 110a is provided in a region where the washing liquid introduction channel 107 and the circulation channel 105 are connected. The switching valve 110a is used to open and close the washing liquid introduction channel 107. The switching valve 110b is provided in the label introduction channel 106. The switching valve 110b is provided in a region where the label introduction channel 106 and the circulation channel 105 are connected. The switching valve 110b is used to open and close the label introduction channel 106. In this embodiment, as shown in FIG. 2, the washing liquid introduction channel 107 and the label introduction channel 106 are connected to the circulation channel 105 through the same connection region (hereinafter, the first connection region).

The switching valve 110g is provided in the sensor channel 109. As shown in FIG. 2, the switching valve 110g is provided in a region where the sensor channel 109 and the circulation channel 105 are connected. The switching valve 110g is used to open and close the sensor channel 109. The switching valve 110h is provided in the waste liquid channel 108. The switching valve 110h is provided in a region where the waste liquid channel 108 and the circulation channel 105 are connected. The switching valve 110h is used to open and close the waste liquid channel 108. In this embodiment, as shown in FIG. 2, the sensor channel 109 and the waste liquid channel 108 are connected to the circulation channel 105 through the same connection region (hereinafter, referred to as the second connection region).

The switching valve 110c, 110d, 110e, 110f is provided in the circulation channel 105. In this embodiment, as shown in FIG. 2, the switching valve 110c is provided adjacent to the first connection region, between the first connection region and the pump 111. The switching valve 110d is provided adjacent to the first connection region, between the first connection region and the air trap 112. The switching valve 110e is provided adjacent to the second connection region, between the second connection region and the pump 111. The switching valve 110f is provided adjacent to the second connection region, between the second connection region and the air trap 112. When all of the switching valves 110c, 110d, 110e, 110f are open, the liquid can circulate within the circulation channel 105.

The pump 111 is used to circulate the liquid in the circulation channel 105. The pump 111 may be a ring pump, a syringe pump, or the like. When a ring pump is used, the liquid in the circulation channel 105 can be circulated by sequentially shifting the position at which the silicon tube of the circulation channel 105 is compressed. When a syringe pump is used, the liquid in the circulation channel 105 can be circulated by sucking in and discharging the liquid in the circulation channel 105 by the action of a piston of the syringe pump. The pump 111 may be inserted in the middle of the circulation channel 105 to circulate the liquid within the circulation channel 105 by sucking in and discharging the liquid. The circulation direction in which the pump 111 circulates the liquid in the circulation channel 105 may be one way, or may be two ways (reciprocating). The process of circulating the liquid in the circulation channel 105 corresponds to a circulation process. The pump 111 may be driven under the control of, for example, a microcomputer, an IC, or the like. The pump 111 may be controlled by the assay device 20 via a communication path that is electrically connected when the cartridge 10 is attached to the assay device 20.

The air trap 112 is a bubble remover that removes or captures bubbles from the liquid in the circulation channel 105 by utilizing a difference in properties between liquid and gas. The process of removing or trapping bubbles from the liquid by the air trap 112 corresponds to a removal process. The air trap 112 and the circulation channel 105 correspond to a pretreatment unit 100 where pretreatment for detecting target molecules is performed. The pretreatment includes binding of the target molecule to the detection material, and washing in which unreacted substances that have not been bound are separated with a washing solution. The pretreatment is performed in the pretreatment process. A target molecule testing method includes the pretreatment process with the circulation process and the removal process. In this embodiment, the detection material is the target-beads complex and the aptamer-enzyme fusion.

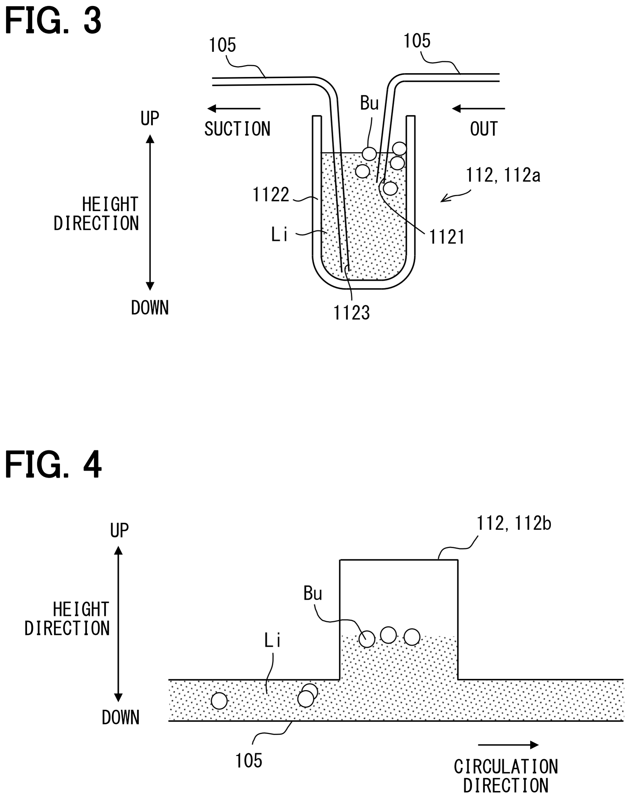

As the mechanism of the air trap 112, the following first to sixths mechanism examples can be given. The mechanism examples will be described below with reference to FIGS. 3 to 8, in which Li indicates the liquid flowing through the circulation channel 105, and Bu indicates the bubbles. In the following, the air trap 112 of the first mechanism example is referred to as an air trap 112a. The air trap 112 of the second mechanism example is referred to as an air trap 112b. The air trap 112 of the third mechanism example is referred to as an air trap 112c. The air trap 112 of the fourth mechanism example is referred to as an air trap 112d. The air trap 112 of the fifth mechanism example is referred to as an air trap 112e. The air trap 112 of the sixth mechanism example is referred to as an air trap 112f. When there is no need to distinguish between the air traps 112a to 112f, they will be referred to as the air trap 112.

As shown in FIG. 3, in the first mechanical example, the air trap 112a has an outlet 1121, a recess 1122, and an inlet 1123. The liquid circulating within the circulation channel 105 flows out from the outlet 1121. The outlet 1121 may be a part of the circulation channel 105. The recess 1122 receives and stores the liquid that flows out of the outlet 1121. The recess 1122 may be, for example, a resin container having a recess for storing liquid. The liquid accumulated in the recess 1122 flows into the circulation channel 105 through the inlet 1123. The inlet 1123 may be a part of the circulation channel 105.

As shown in FIG. 3, in the air trap 112a, the outlet 1121 is located higher than the inlet 1123 in the height direction. This allows the air trap 112a to remove bubbles from the liquid in the circulation channel 105 by utilizing a difference in density between the liquid and the bubbles. The details will be described as follows. Since the air bubbles have a lower density than the liquid, the air bubbles move vertically upwards relative to the liquid. Therefore, after the air bubbles are discharged from the outlet 1121 of the circulation channel 105, the air bubbles do not head toward the inlet 1123 but head upward in the vertical direction. As a result, the air bubbles in the liquid are removed from the circulation channel 105 by the air trap 112a.

The number of the air traps 112a provided for the circulation channel 105 is not limited to one. Plural air traps 112a may be provided for the circulation channel 105. In other words, the air trap 112a may have one or more sets of the outlet 1121, the recess 1122, and the inlet 1123.

As shown in FIG. 4, in the second mechanical example, the air trap 112b has a chamber structure in which a part of the circulation channel 105 is bulged upward in the height direction, The air trap 112b can capture air bubbles mixed in the liquid in the circulation channel 105 by utilizing a difference in density between the liquid and the air bubbles. The details will be described as follows.

Since the air bubbles have a lower density than the liquid, the air bubbles move vertically upwards relative to the liquid. Therefore, the air bubbles of the liquid in the circulation channel 105 are directed toward the air trap 112b having the chamber structure. As a result, the air bubbles are captured in the air trap 112b and do not circulate through the circulation channel 105. The air trap 112b is not limited to being provided one for the circulation channel 105. For example, plural air traps 112b may be provided in the circulation channel 105.

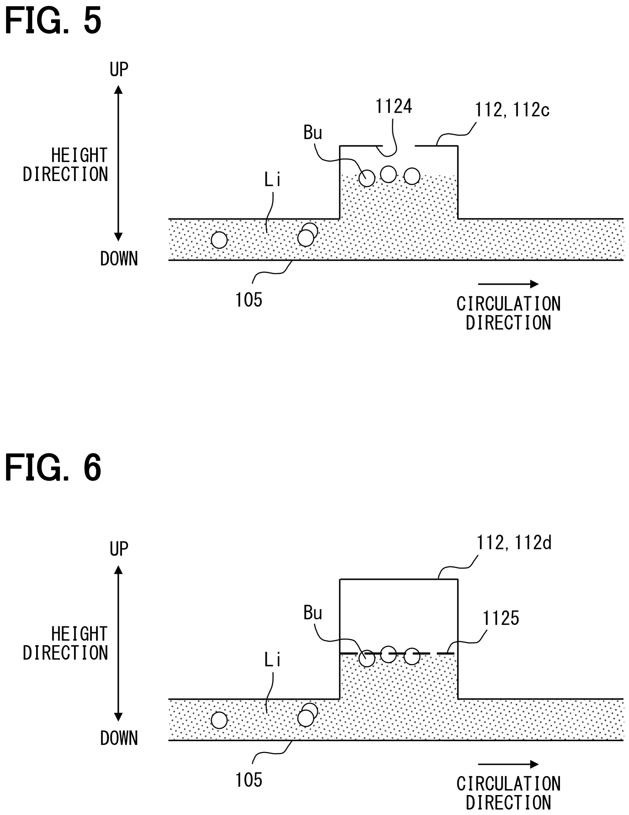

As shown in FIG. 5, in the third mechanical example, the air trap 112c has a chamber structure in which a part of the circulation channel 105 is bulged upward in the height direction. The air trap 112c has a bubble outlet 1124 at the upper side in the height direction of the chamber structure. The air trap 112c can remove air bubbles from the liquid by utilizing a difference in density between the liquid and the air bubbles. The details will be described as follows.

Since the air bubbles have a lower density than the liquid, the air bubbles move vertically upwards relative to the liquid. Therefore, the air bubbles are directed toward the air trap 112c having the chamber structure from the liquid introduced into the circulation channel 105. The air bubbles that have reached the air trap 112c are released to the outside of the circulation channel 105 through the outlet 1124. As a result, air bubbles are removed from the liquid in the circulation channel 105 by the air trap 112c. Furthermore, according to the third mechanical example, since the air bubbles are released from the outlet 1124 of the chamber structure, it is not necessary to continuously capture the air bubbles in the chamber structure. This allows the chamber structure to be miniaturized. The air trap 112c is not limited to being provided one for the circulation channel 105. For example, plural air traps 112c may be provided in the circulation channel 105.

In the fourth mechanical example, as shown in FIG. 6, the air trap 112d has a chamber structure in which a part of the circulation channel 105 is bulged upward in the height direction. In the air trap 112d, the chamber structure is further provided with a permeable membrane 1125 that divides the space in the height direction. The permeable membrane 1125 has selective permeability that does not allow the liquid flowing through the circulation channel 105 to pass therethrough but allows air bubbles to pass therethrough. The permeable membrane 1125 may be in the form of a gel. In addition, the upper space of the chamber structure upper than the permeable membrane 1125 in the height direction is a gas layer. The air trap 112d can capture air bubbles from the liquid by utilizing the difference in density between the liquid and the air bubbles. The details will be described as follows.

Since the air bubbles have a lower density than the liquid, the air bubbles move vertically upwards relative to the liquid. Therefore, the air bubbles are directed toward the air trap 112d having the chamber structure. The air bubbles heading toward the air trap 112c pass through the permeable membrane 1125 and are captured in the gas layer of the chamber structure. As a result, the air bubbles are captured in the air trap 112d and do not circulate through the circulation channel 105. The air trap 112d is not limited to being provided for the circulation channel 105. For example, plural air traps 112d may be provided in the circulation channel 105. In addition, the fourth and third mechanical examples may be combined. In other words, an outlet for air bubbles may be provided on the upper side in the height direction of the chamber structure of the air trap 112d.

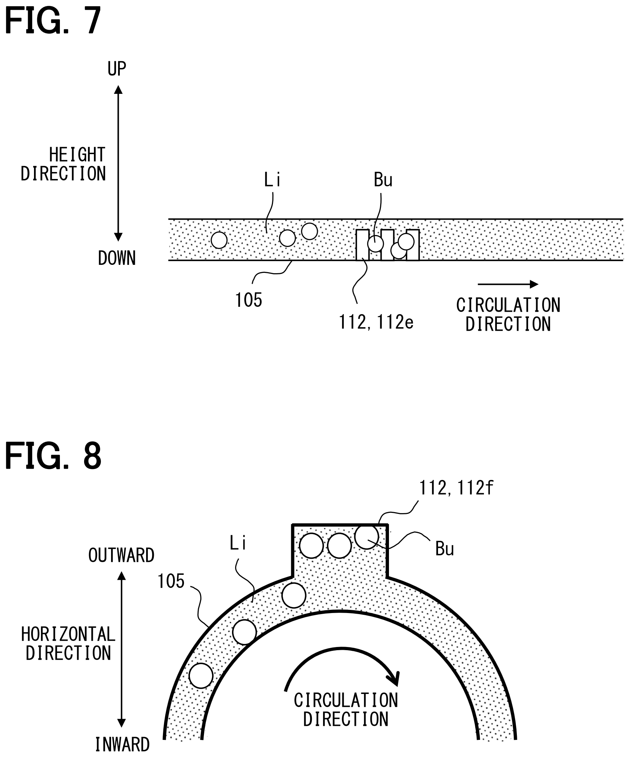

In the fifth mechanical example, as shown in FIG. 7, the air trap 112e includes plural columnar structures having irregularities on the surface, inside the circulation channel 105. The columnar structures protrude from the inner wall of the circulation channel 105. The air trap 112e is capable of capturing air bubbles from the liquid flowing in the circulation channel 105 by utilizing the difference in properties between the liquid and the air bubbles. The details will be described as follows.

A surface tension is generated in the columnar structures inside the circulation channel 105 by the liquid flowing through the circulation channel 105. The surface of liquid has the property of capturing gas molecules, so the surface tension generated in the columnar structures makes it possible to capture the bubbles. In the fifth mechanism example, the surface of the columnar structure has projections and recesses, so that the surface tension is more likely to be generated. In the fifth mechanism example, since the plural columnar structures are provided, it becomes easier to capture a larger number of bubbles. The unevenness on the surface of the columnar structure is preferably provided with plural fine unevenness to facilitate generation of surface tension. It is preferable that plural columnar structures are provided within a predetermined range not to be too far apart from each other. Accordingly, the air bubbles are trapped between the columnar structures, making it easier for the air bubbles to continue to be trapped by the columnar structures. The predetermined range may be set arbitrarily.

In the sixth mechanism example, as shown in FIG. 8, at least a part of the circulation channel 105 has an arc shape. In the sixth mechanical example, as shown in FIG. 8, the air trap 112f has a chamber structure in which a part of the circulation channel 105 bulges outward from the outer periphery of the arc-shaped portion of the circulation channel 105. The air trap 112f can remove air bubbles from the liquid in the circulation channel 105 by utilizing the difference in density between the liquid and the air bubbles. The details will be described as follows.

In the arc-shaped portion of the circulation channel 105, a centrifugal force is generated in the fluid toward the outer periphery. Furthermore, air bubbles, which have a lower density than the liquid, are pushed toward the outer periphery by the centrifugal force generated in the liquid, and are captured in the chamber structure of the sixth mechanism example. As a result, air bubbles are captured in the air trap 112f and do not circulate through the circulation channel 105. The air trap 112f is not limited to being provided for the circulation channel 105. Plural air traps 112f may be provided in the circulation channel 105.

As shown in FIG. 2, the magnetic unit 113 holds the antibody-attached magnetic beads in the liquid flowing in the circulation channel 105 in a specific region of the circulation channel 105 by magnetic force. The magnetic unit 113 may be provided, outside the circulation channel 105, in the vicinity of a specific region of the circulation channel 105. The specific region may be arbitrarily set. The specific region may be, for example, between the second connection region and the pump 111. In this case, the magnetic unit 113 may hold or release the antibody-attached magnetic beads in a specific region of the circulation channel 105 by, for example, moving a magnet toward or away from the circulation channel 105. In this case, a drive device may be used to control the distance between the circulation channel 105 and the magnet. Alternatively, the magnetic unit 113 may hold or release the antibody-attached magnetic beads in a specific region of the circulation channel 105 by, for example, intermittently generating a magnetic force in an electromagnet. In this case, the magnetic force of the electromagnet can be intermittently generated by passing or stopping a current through the electromagnet. The magnetic unit 113 may be driven under the control of, for example, a microcomputer, an IC, or the like. The magnetic unit 113 may be controlled by the assay device 20 via a communication path that is electrically connected when the cartridge 10 is attached to the assay device 20.

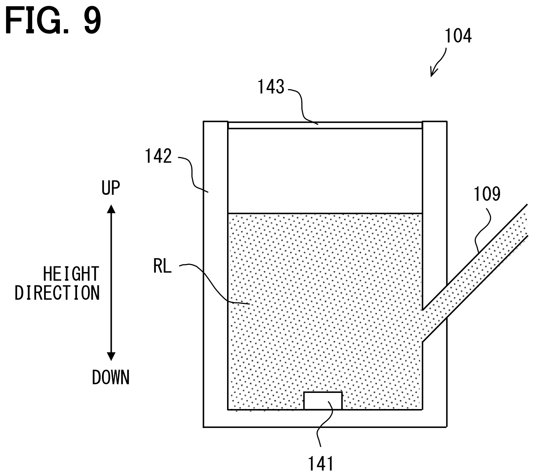

Next, a schematic configuration of the sensor area 104 will be described with reference to FIG. 9. As shown in FIG. 9, the sensor area 104 of the present embodiment includes a sensor chip 141, a sensor container 142, and a diaphragm 143.

The sensor chip 141 performs sensing based on a substance produced by the reaction between the aptamer-enzyme fusion of the sandwich complex and the reaction liquid. Specifically, sensing is performed based on a substance produced by the reaction between the enzyme of the aptamer-enzyme fusion and the reaction liquid. The sensor chip 141 corresponds to a sensor. In this embodiment, sensing is performed based on hydrogen ions as a detection signal generated by the reaction between ALP and p-nitrophenyl phosphate. The sensor chip 141 may be a hydrogen ion sensor for measuring the concentration of hydrogen ions. The hydrogen ion sensor may be a semiconductor sensor that detects hydrogen ions by utilizing a metal oxide semiconductor. The sensing results of the sensor chip 141 may be transmitted to the assay device 20 via a communication path that is electrically connected when the cartridge 10 is attached to the assay device 20. In the hydrogen ion sensor, a voltage corresponding to the concentration of hydrogen ions is obtained as the sensing result. In the assay device 20, the presence of the target molecule may be detected based on the sensing result in the sensor chip 141, and the amount of the target molecule may be quantified.

It is possible to determine whether or not the target molecule is present in a sample using the testing system 1 by inspecting the sample, in which it is uncertain whether or not a target molecule is present. For example, when the target molecule is a pathogen or a part thereof, it becomes possible to determine the presence or absence of the pathogen in a sample. This makes it suitable for use in testing for the presence or absence of pathogen infection in the human body.

The sensor chip 141 is provided in the sensor container 142. The sensor container 142 is made of, for example, resin having a depression for storing liquid. As shown in FIG. 9, the sensor container 142 holds a reaction liquid RL. The sensor chip 141 is provided in the sensor container 142 so as to be immersed in the reaction liquid RL. As a specific example, as shown in FIG. 9, the sensor chip 141 is provided on the lower side in the height direction of the sensor container 142. As shown in FIG. 9, the sensor container 142 is connected to the sensor channel 109, and the reaction liquid RL can be introduced and discharged between the sensor container 142 and the circulation channel 105 via the sensor channel 109. As shown in FIG. 9, the sensor container 142 has an opening on the upper side in the height direction.

The diaphragm 143 is provided to close the upper opening of the sensor container 142. The diaphragm 143 functions as a controller mechanism for introducing/discharging the reaction liquid RL sealed in the sensor container 142 to/from the circulation channel 105. The diaphragm 143 allows the reaction liquid RL sealed in the sensor container 142 to flow in and out of the circulation channel 105 via the sensor channel 109. As a result, the sandwich complex obtained by the binding and washing is drawn from the circulation channel 105 into the sensor container 142. The process of controlling the flow of the reaction liquid RL by the controller mechanism and the process of drawing in of the sandwich complex correspond to a control process. The diaphragm 143 is an elastic membrane which is metallic or non-metallic. According to the above configuration, the reaction liquid RL is introduced into and discharged from the circulation channel 105, whereby the detection target can be brought from the circulation channel 105 into the sensor container 142. Therefore, it is no longer necessary to use a separate carrier liquid for carrying the detection target into the sensor container 142. As a result, the reaction liquid RL is not diluted with the carrier liquid, and it is possible to restrict a decrease in the detection sensitivity of the detection target. The diaphragm 143 may be driven hydraulically or electrically. The diaphragm 143 may be driven under the control of, for example, a microcomputer, an IC, or the like. The diaphragm 143 may be controlled by the assay device 20 via a communication path that is electrically connected when the cartridge 10 is attached to the assay device 20.

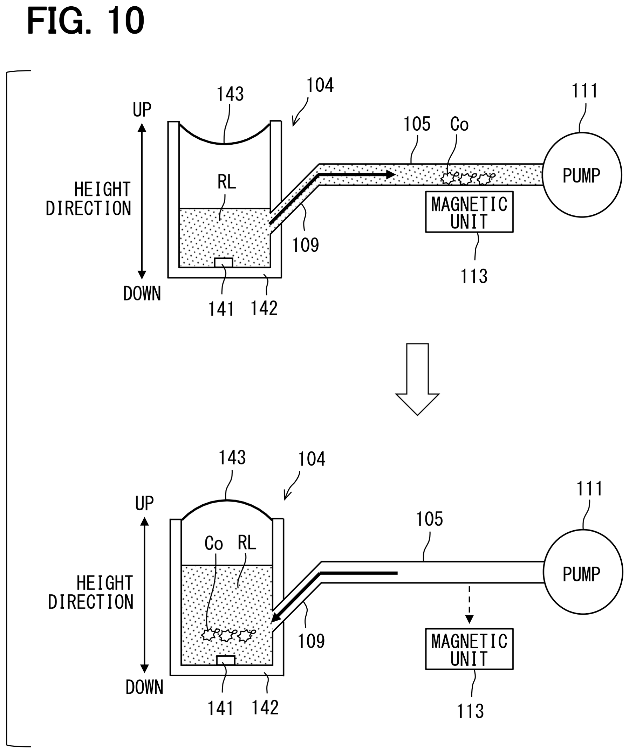

The function of the diaphragm 143 will be described with reference to FIG. 10. In FIG. 10, the switching valve 110e, 110g is omitted for simplicity. The sandwich complex is represented by Co in FIG. 10. FIG. 10 shows the flow of the reaction liquid RL in response to the change in the diaphragm 143. As shown in the upper side of FIG. 10, the sandwich complex Co is held in a specific region in the circulation channel 105 by the magnetic force of the magnetic unit 113. The sandwich complex Co is in a state where the above-mentioned bonding and washing have been completed. Hereinafter, the process in which the diaphragm 143 is pressed downward in the height direction will be referred to as the discharge process. The process in which the diaphragm 143 is pulled upward in the height direction is referred to as the suction process.

As shown in FIG. 10, in the discharge process, the diaphragm 143 bends downward, causing the pressure inside the sensor container 142 to increase. As a result, the reaction liquid RL sealed in the sensor container 142 flows out to the sensor channel 109. The flow of the reaction liquid RL passes through the sensor channel 109 and reaches the region in the circulation channel 105 where the sandwich complex Co is held. In the discharge process, the switching valve 110e, 110g is opened. The holding of the sandwich complex Co by the magnetic force of the magnetic unit 113 is released when moving to the suction process.

As shown in the lower side of FIG. 10, in the suction process, the diaphragm 143 is deflected upward, causing the pressure inside sensor container 142 to decrease. As a result, the reaction liquid RL that has flowed out from the sensor container 142 to the circulation channel 105 is sucked into the sensor container 142. At this time, the sandwich complex Co is also sucked into the sensor container 142 from the circulation channel 105 together with the reaction liquid RL. In the sensor container 142, the sensor chip 141 performs sensing based on a substance produced by a reaction between the enzyme contained in the sandwich complex Co and the reaction liquid RL.

It is preferable that the volume of the sensor container 142 changes according to the change in the amount of the reaction liquid remaining in the sensor container 142 when the reaction liquid sealed in the sensor container 142 is introduced into and discharged from the circulation channel 105. This makes it possible to quickly suppress pressure fluctuations inside the sensor container 142 and reduce noise such as the intrusion of external air caused by negative pressure. Such a configuration can be achieved by using the diaphragm 143 as the controller mechanism.

In the present embodiment, the diaphragm 143 is used as the controller mechanism, but not limited to this. For example, a syringe may be used as the controller mechanism. When a syringe is used, it becomes possible to change the volume of the sensor container 142 in response to a change in the amount of the reaction liquid remaining in the sensor container 142.

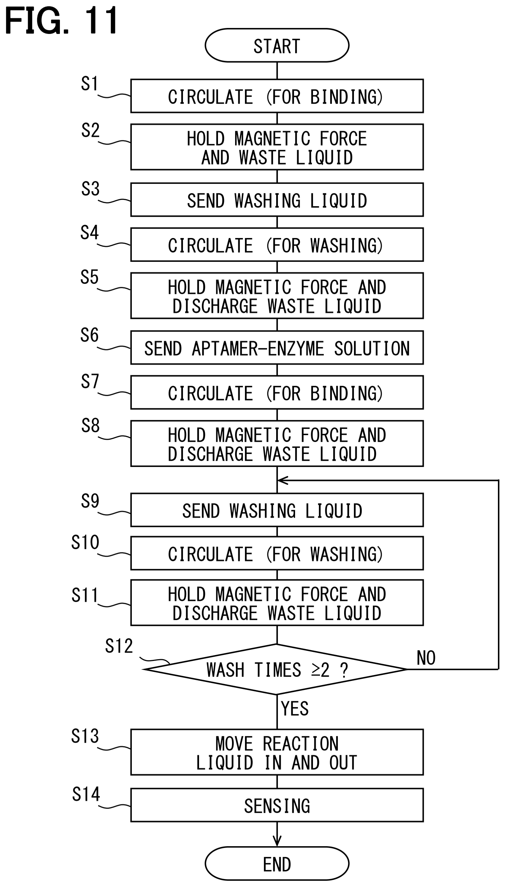

A flow of processing related to the detection of target molecule in the cartridge 10 (hereinafter, detection-related processing) will be described with reference to the flowchart of FIG. 11. The flowchart of FIG. 11 may start, for example, when a sample containing a target molecule and antibody-attached magnetic beads (hereinafter, a mixed liquid) is sent into the circulation channel 105. When the mixed liquid is sent into the circulation channel 105, the switching valve 110c, 110d, 110e, 110f is open, and the switching valve 110a, 110b, 110g, 110h is closed. In the flowchart of FIG. 11, the explanation will be continued by taking as an example a case where a target molecule is present in a sample. The amount of the mixed liquid may be adjusted by adding a buffer or the like so as to reach the specified amount. The mixed liquid may be introduced into the circulation channel 105 from an inlet provided in the circulation channel 105. The air trap 112 may be used as the inlet. In this case, for example, as in the third mechanism example, an air trap 112 capable of introducing liquid into the circulation channel 105 from outside the circulation channel 105 may be used.

In the flowchart of FIG. 11, for example, receipt of an input to start a testing in the assay device 20 may also be included as a start condition. In the following description, the assay device 20 controls the members of the cartridge 10 subject to drive control. In the flow chart of FIG. 11, the explanation will be continued on the assumption that the measurement of the sensor drift has already been started in the sensor area 104 in which the reaction liquid is sealed.

In step S1, the pump 111 is driven to circulate the mixed liquid within the circulation channel 105. During this circulation, a target molecule contained in the sample binds to the antibody-attached magnetic beads. Through this binding, the target molecule and the antibody-attached magnetic beads form a target-beads complex. In S1, the switching valve 110c, 110d, 110e, 110f is open, and the switching valve 110a, 110b, 110g, 110h is closed.

In step S2, the antibody-attached magnetic beads are held in a specific region of the circulation channel 105 by the magnetic force of the magnetic unit 113. As a result, the target-beads complex containing the antibody-attached magnetic beads is also retained in a specific region of the circulation channel 105. Then, in S2, the mixed liquid is discharged as waste liquid into the waste liquid area 103 via the waste liquid channel 108. The target-beads complex is held in a specific region of the circulation channel 105 by magnetic force, so that the discharged mixed liquid does not contain the target-beads complex. When the mixed liquid is discharged to the waste liquid area 103, the switching valve 110h is opened. After the discharge, the switching valve 110h is closed. The mixed liquid may be discharged to the waste liquid area 103 by driving the pump 111.

In step S3, the washing liquid is sent from the washing liquid holding area 102 to the circulation channel 105 via the washing liquid introduction channel 107. When the washing liquid is delivered, the switching valve 110a is opened. After the liquid is delivered, the switching valve 110a is closed. The washing liquid may be sent to the circulation channel 105 by driving the pump 111. The amount of the washing liquid sent to the circulation channel 105 may be a specified amount. The amount of the washing liquid sent to the circulation channel 105 may be adjusted by, for example, adjusting the timing of opening and closing the switching valve 110a.

In step S4, the pump 111 is driven to circulate the washing liquid within the circulation channel 105. This circulation washes the target-beads complex and separates unreacted materials from the target-beads complex. The washing can be performed after the target-beads complex is released from the magnetic force of the magnetic unit 113. The washing may be performed while the target-beads complex is being continuously held by the magnetic force of the magnetic unit 113. In S4, the switching valve 110c, 110d, 110e, 110f is open, and the switching valve 110a, 110b, 110g, 110h is closed.

In step S5, the target-beads complex is held in a specific region of the circulation channel 105 by the magnetic force of the magnetic unit 113. In S5, the washing liquid used for cleaning is discharged as waste liquid into the waste liquid area 103 via the waste liquid channel 108. The target-beads complex is held in a specific region of the circulation channel 105 by magnetic force, so that the discharged washing liquid does not contain the target-beads complex. When the washing liquid is discharged to the waste liquid area 103, the switching valve 110h is opened. After the discharge, the switching valve 110h is closed. The washing liquid can be discharged to the waste liquid area 103 by driving the pump 111.

In step S6, a buffer containing the aptamer-enzyme fusion (hereinafter, referred to as an aptamer-enzyme solution) is sent from the label holding area 101 to the circulation channel 105 via the label introduction channel 106. In the circulation channel 105 to which the aptamer-enzyme solution is delivered, the target-beads complex is held by the magnetic force of the magnetic unit 113. The amount of the aptamer-enzyme solution is a specified amount. When the aptamer-enzyme solution is delivered, the switching valve 110b is opened. After the solution is delivered, the switching valve 110b is closed. The aptamer-enzyme solution can be sent to the circulation channel 105 by driving the pump 111.

In step S7, the pump 111 is driven to circulate the aptamer-enzyme solution and the target-beads complex within the circulation channel 105. This circulation is performed after the target-beads complex is released from the magnetic force of magnetic unit 113. During this circulation, the aptamer-enzyme fusion and the target-beads complex are bound to each other. This binding results in the sandwich complex between the aptamer-enzyme fusion and the target-beads complex. In S7, the switching valve 110c, 110d, 110e, 110f is open, and the switching valve 110a, 110b, 110g, 110h is closed.

In step S8, the antibody-attached magnetic beads are held in a specific region of the circulation channel 105 by the magnetic force of the magnetic unit 113. As a result, the sandwich complex containing the antibody-attached magnetic beads is also retained in a specific region of the circulation channel 105. Then, in S8, the aptamer-enzyme solution is discharged as waste liquid into the waste liquid area 103 via the waste liquid channel 108. Since the sandwich complex is held in a specific region of the circulation channel 105 by magnetic force, the sandwich complex is not contained in the discharged aptamer-enzyme solution. When the aptamer-enzyme solution is discharged to the waste liquid area 103, the switching valve 110h is opened. After the discharge, the switching valve 110h is closed. The aptamer-enzyme solution can be discharged to the waste liquid area 103 by driving the pump 111.

In step S9, similarly to S4, the washing liquid is sent from the washing liquid holding area 102 to the circulation channel 105 via the washing liquid introduction channel 107. In step S10, the pump 111 is driven to circulate the washing liquid within the circulation channel 105, in the same manner as in step S5. This circulation washes the sandwich complex and separates unreacted substances from the sandwich complex. In step S11, the sandwich complex is held in a specific region of the circulation channel 105 by the magnetic force of the magnetic unit 113. Then, in S11, the washing liquid used for cleaning is discharged as waste liquid into the waste liquid area 103 via the waste liquid channel 108. Since the sandwich complex is held in a specific region of the circulation channel 105 by magnetic force, the sandwich complex is not contained in the discharged washing liquid.

In step S12, if the sandwich complex has been washed two or more times (YES in S12), the process proceeds to step S13. If the sandwich complex has been washed less than twice (NO in S12), the process proceeds to S9.

In step S13, the diaphragm 143 is driven to move the reaction liquid sealed in the sensor container 142 into and out of the circulation channel 105. In S13, the switching valve 110g is opened and the switching valve 110h is closed. In S13, the diaphragm 143 is driven in order of the discharge stroke and the suction stroke. In the discharge stroke, the reaction liquid sealed in the sensor container 142 is sent through the sensor channel 109 to the region in the circulation channel 105 where the sandwich complex is held. In the suction stroke, the sandwich complex is released from the magnetic force of the magnetic unit 113. Then, the reaction liquid that has flowed out from the sensor container 142 to the circulation channel 105 is sucked into the sensor container 142. At this time, the sandwich complex is also sucked into the sensor container 142 together with the reaction liquid.

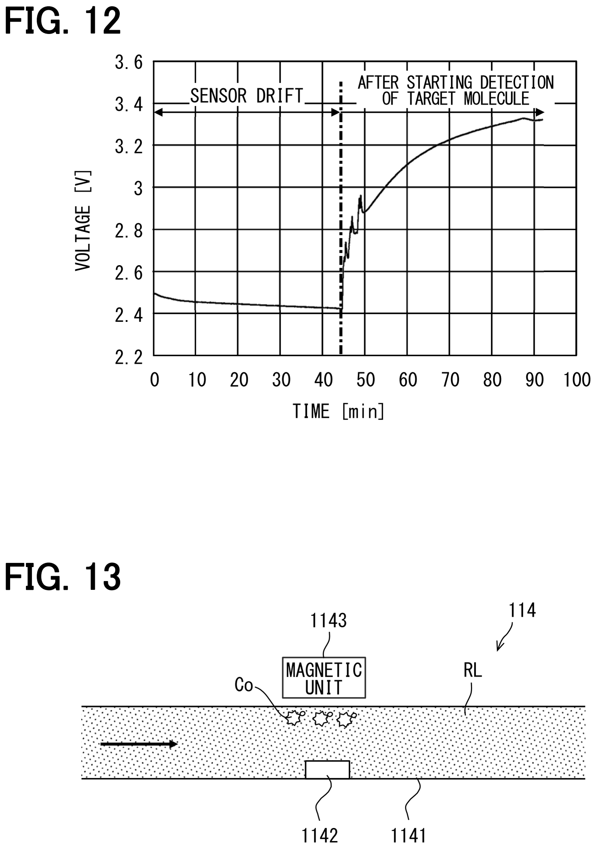

In step S14, the sensor chip 141 performs sensing based on a substance generated by the reaction between the enzyme contained in the sandwich complex and the reaction liquid. In this embodiment, a voltage is sensed, which corresponds to the concentration of hydrogen ions generated by hydrolysis reaction of p-nitrophenyl phosphate and water using ALP as a catalyst. In S14, the sensor chip 141 may output the ratio to the voltage measured during the sensor drift as the sensing result (see FIG. 12). FIG. 12 is a diagram showing a change over time in the voltage measured by the sensor chip 141. As shown in FIG. 12, the voltage is measured during the stage of sensor drift. Therefore, the sensor chip 141 improves the measurement accuracy by outputting the ratio to the voltage measured due to the sensor drift as the sensing result. In the assay device 20, the amount of the target molecule can be quantified based on the sensing result in the sensor chip 141.

In the flowchart of FIG. 11, the washing in S4 is performed once, as an example, but not necessarily limited to this. For example, washing may be repeated multiple times, similar to the washing in S10.

According to the first embodiment, the binding of the target molecule to the target-beads complex and the aptamer-enzyme fusion, and the washing of unreacted substances are performed in the circulation channel 105. Since the liquid is circulated in the circulation channel 105, the target molecule, the target-bead complex, and the aptamer-enzyme fusion are more easily mixed in the circulation channel 105. Thus, there is a higher chance of the target molecule binding to the target-beads complex and the aptamer-enzyme fusion. Therefore, it becomes possible to increase the ratio of the target molecule that can bind to the target-beads complex and the aptamer-enzyme fusion. The circulation channel 105 is tubular, and air bubbles entrained in the liquid within the circulation channel 105 may circulate within the circulation channel 105 together with the target molecule, the target-beads complex and the aptamer-enzyme fusion. In contrast, according to the first embodiment, the air trap 112 makes it possible to remove or capture bubbles from the liquid. Thus, the air trap 112 reduces the likelihood of bubbles remaining in contact with the target molecule, the target-beads complex, and the aptamer-enzyme fusion, which can inhibit the binding and the washing. As a result, even when the binding and the washing of the target molecule and the binding target are performed within a channel, it is possible to facilitate the binding of the target molecule within the channel while reducing the hindrance of binding and washing of the target molecule due to bubbles entering the channel.

### (Second Embodiment)

In the first embodiment, a controller mechanism is used in the sensor area 104, but not necessarily limited to this. A second embodiment is described with reference to FIG. 13. The testing system 1 of the second embodiment is similar to the testing system 1 of the first embodiment, except that the cartridge 10 has a sensor area 114 instead of the sensor area 104.

The sensor area 114 is similar to the sensor area 104 of the first embodiment, except for some differences. Hereinafter, the differences will be described. A schematic configuration of the sensor area 114 will be described with reference to FIG. 13. As shown in FIG. 13, the sensor area 114 includes a passage 1141, a sensor chip 1142, and a magnetic unit 1143.

The sensor chip 1142 is provided in the passage 1141. The passage 1141 may be made of, for example, a tubular resin. The passage 1141 may be a silicon tube. The passage 1141 may be formed in a loop shape such that the liquid flowing from the sensor channel 109 returns to the sensor channel 109. The passage 1141 may be connected to a container for holding the reaction liquid described in the first embodiment (hereinafter, reaction liquid holding container). The connection between the reaction liquid holding container and the passage 1141 may be switched by a valve similar to the switching valve 110.

The sensor chip 1142 performs sensing in the same manner as the sensor chip 141 of the first embodiment. The sensor chip 1142 is provided in the passage 1141. The magnetic unit 1143 uses magnetic force to hold the antibody-attached magnetic beads in the liquid flowing inside the passage 1141 near the sensor chip 1142 in the passage 1141. The magnetic unit 1143 may be provided outside the passage 1141 and near the sensor chip 1142. The magnetic unit 1143 may hold and release the antibody-attached magnetic beads in the same manner as the magnetic unit 113.

In the second embodiment, instead of step S13 in the flowchart of FIG. 11 in the first embodiment, for example, the following process may be performed. First, a carrier liquid is sent from the circulation channel 105 through the sensor channel 109 to the passage 1141. As the carrier liquid, for example, the washing liquid from the washing liquid holding area 102 may be used. This liquid transfer is performed after the sandwich complex held by the magnetic force of the magnetic unit 113 is released. During this liquid transfer, the sandwich complex is held in the vicinity of the sensor chip 1142 within the passage 1141 by the magnetic unit 1143. The sandwich complex held near the sensor chip 1142 is represented by Co in FIG. 13.

Next, the reaction liquid is sent from the reaction liquid holding container to the passage 1141. This liquid transfer is performed after the sandwich complex held by the magnetic force of the magnetic unit 113 is released. By sending this reaction liquid (see RL in FIG. 13), the carrier liquid is sent out from the passage 1141, and the liquid in the vicinity of the sensor chip 1142 is replaced with the reaction liquid. In the passage 1141, the sensor chip 1142 performs sensing based on a substance produced by a reaction between the enzyme contained in the sandwich complex Co and the reaction liquid RL. The carrier liquid sent out from the passage 1141 may be discharged to the waste liquid area 103 via, for example, the sensor channel 109 and the waste liquid channel 108. In this case, the switching valve 110e, 110f is closed, and the switching valve 110g, 110h is opened.

### (Third Embodiment)

In the above embodiment, the cartridge 10 is detachable from the assay device 20, but not necessarily limited to this. For example, the functions performed by the components of the cartridge 10 may be performed by the assay device 20. Furthermore, some of the functions performed by the components of the cartridge 10 may be performed by the assay device 20. For example, the functions of the sensor area 104, 114 may be performed by the assay device 20 instead of the cartridge 10.

This description discloses plural technical ideas set forth in the following. Some clauses are in a multiple dependent form, where the subsequent clause refers to the preceding clause as an alternative. Some clauses are in a multiple dependent form referring to another multiple dependent form. These sections written in the multiple dependent form define plural technical ideas.

In a first aspect, an assay device configured to detect a target molecule, includes: a pretreatment unit 100 having a circulation channel 105 which is a tubular passage through which a liquid circulates, in which the target molecule is bound with a detection material for detecting the target molecule and a washing is performed with a washing solution to separate unreacted substance that is not bound as a pretreatment for detecting the target molecule; and a bubble remover 112, 112a, 112b, 112c, 112d, 112e, 112f configured to remove or capture bubbles mixed in the liquid circulating in the circulation channel by utilizing a difference in properties between liquid and gas.

According to a second aspect, in the assay device of the first aspect, a sensor 141 performs sensing based on a substance generated by a reaction between the detection material and a reaction liquid. A sensor container 142 houses the sensor 141. A controller mechanism 143 is configured to control the reaction liquid sealed in the sensor container to flow into and from the circulation channel. The controller mechanism draws a complex of the target molecule and the detection material bound with each other, after the washing, from the circulation channel into the sensor container by controlling the reaction liquid sealed in the sensor container to flow into and from the circulation channel.

According to a third aspect, in the assay device of the second aspect, a volume of the sensor container changes in accordance with a change in amount of the reaction liquid remaining in the sensor container when the reaction liquid sealed in the sensor container is controlled to flow into or out of the circulation channel by the controller mechanism.

According to a fourth aspect, in the assay device of any one of the first to third aspects, the bubble remover 112a has at least one set of an outlet 1121 through which the liquid circulating in the circulation channel flows out, a recess 1122 to receive and store the liquid flowing out of the outlet, and an inlet 1123 to allow the liquid stored in the recess to flow into the circulation channel. In the set of the outlet, the recess, and the inlet, the outlet is located higher in a height direction than the inlet.

According to a fifth aspect, in the assay device of any one of the first to third aspects, at least a part of the circulation channel has an arc shape, and the bubble remover 112f has a chamber structure in which a part of the circulation channel is bulged outwardly of the arc shape of the circulation channel.

According to a sixth aspect, in the assay device of any one of the first to third aspects, the bubble remover 112e includes plural columnar structures having irregularities on the surface, inside the circulation channel.

According to a seventh aspect, in the assay device of any one of the first to third aspects, the bubble remover 112b, 112c, 112d has a chamber structure in which a part of the circulation channel is bulged upward in the height direction.

According to an eighth aspect, in the assay device of the seventh aspect, the chamber structure has an air bubble outlet 1124 on an upper side in the height direction.

According to a ninth aspect, in the assay device of the seventh or eighth aspect, the chamber structure includes a permeable membrane 1125 having selective permeability not to allow the liquid to pass through but allows the bubbles to pass through. The permeable membrane divides a space of the chamber structure in the height direction, and an upper space of the chamber structure upper than the permeable membrane in the height direction has a gas layer.

The present disclosure is not limited to the embodiments, and various modifications are possible within the scope of the claims. An embodiment obtained by appropriately combining technical means disclosed in different embodiments is also included in the technical scope of the present disclosure.

## Claims

1. An assay device configured to detect a target molecule, the assay device comprising:
a pretreatment unit (100) having a circulation channel (105) which is a tubular passage through which a liquid circulates, in which the target molecule is bound with a detection material for detecting the target molecule and a cleaning is performed with a washing solution to separate unreacted substance that is not bound, as a pretreatment for detecting the target molecule; and
a bubble remover (112, 112a, 112b, 112c, 112d, 112e, 112f) configured to remove or capture bubbles mixed in the liquid circulating in the circulation channel by utilizing a difference in properties between liquid and gas.

2. The assay device according to claim 1, further comprising:
a sensor (141) configured to perform sensing based on a substance generated by a reaction between the detection material and a reaction liquid;
a sensor container (142) in which the sensor is provided; and
a controller mechanism (143) configured to control the reaction liquid sealed in the sensor container to flow into and out of the circulation channel, wherein
the controller mechanism draws a complex of the target molecule and the detection material bound with each other, after the cleaning, from the circulation channel into the sensor container by controlling the reaction liquid sealed in the sensor container into and from the circulation channel.

3. The assay device according to claim 2, wherein
a volume of the sensor container changes in accordance with a change in amount of the reaction liquid remaining in the sensor container when the reaction liquid sealed in the sensor container is controlled to flow into or out of the circulation channel by the controller mechanism.

4. The assay device according to any one of claims 1 to 3, wherein
the bubble remover (112a) has at least one set of an outlet (1121) through which the liquid circulating in the circulation channel flows out, a recess (1122) to receive and store the liquid flowing out of the outlet, and an inlet (1123) through which the liquid stored in the recess to flow into the circulation channel, and
the outlet is located higher than the inlet in a height direction, in one set of the outlet, the recess, and the inlet.

5. The assay device according to any one of claims 1 to 3, wherein
at least a part of the circulation channel has an arc shape, and
the bubble remover (112f) has a chamber structure in which a part of the circulation channel is bulged outwardly of the arc shape of the circulation channel.

6. The assay device according to any one of claims 1 to 3, wherein
the bubble remover (112e) includes a plurality of columnar structures having irregularities on a surface, inside the circulation channel.

7. The assay device according to any one of claims 1 to 3, wherein
the bubble remover (112b, 112c, 112d) has a chamber structure in which a part of the circulation channel is bulged upward in a height direction.

8. The assay device according to claim 7, wherein
the chamber structure has an air bubble outlet (1124) on an upper side in the height direction.

9. The assay device according to claim 7 or 8, wherein
the chamber structure includes a permeable membrane (1125) having selective permeability not to allow the liquid to pass through but allows the bubbles to pass through,
the permeable membrane divides a space of the chamber structure in the height direction, and
an upper space of the chamber structure upper than the permeable membrane in the height direction has a gas layer.

10. A target molecule testing method to detect a target molecule, comprising:
binding the target molecule to a detection material for detecting the target molecule, and cleaning with a washing solution to separate unreacted substance that is not bound, as a pretreatment process for detecting the target molecule, wherein
the pretreatment process includes:
circulating a liquid in a circulation channel (105) which is a tubular passage for circulating the liquid for the binding and the cleaning; and
controlling bubbles to be captured or removed from the liquid by a bubble remover (112, 112a, 112b, 112c, 112d, 112e, 112f) by utilizing a difference in properties between liquid and gas.

11. The target molecule testing method according to claim 10, further comprising:
controlling a reaction liquid sealed in a sensor container (142) to flow into or out of the circulation channel by a controller mechanism (143), such that a complex of the target molecule and the detection material, after the cleaning, is drawn from the circulation channel into the sensor container; and
sensing with a sensor (141) housed in the sensor container based on a substance produced by a reaction between the detection material and the reaction liquid.
